**Europäisches Patentamt**

**European Patent Office**

**Office européen des brevets**

(11) Veröffentlichungsnummer: **0 472 001 A2**

(12) **EUROPÄISCHE PATENTANMELDUNG**

(21) Anmeldenummer: **91112550.8**

(22) Anmeldetag: **26.07.91**

(51) Int. Cl.⁵: **G01N 33/00**

(30) Priorität: **23.08.90 DE 4026591**

(43) Veröffentlichungstag der Anmeldung:
**26.02.92 Patentblatt 92/09**

(84) Benannte Vertragsstaaten:
**AT CH DE FR GB IT LI NL**

(71) Anmelder: **PLEVA GMBH**
**Rudolf-Diesel-Strasse 2**
**W-7246 Empfingen(DE)**

(72) Erfinder: **Pleva, Ralf**
**Griesweg 26**
**W-7240 Horb(DE)**

(74) Vertreter: **Jackisch, Walter, Dipl.-Ing. et al**
**Menzelstrasse 40**
**W-7000 Stuttgart 1(DE)**

(54) Verfahren zur Bestimmung der Beladung von Luft mit Dämpfen, insbesondere Wasserdampf.

(57) 2.1 Es ist Aufgabe der Erfindung, ein Verfahren anzugeben, das die Anwendung von Sauerstoffsensoren zur Feuchtemessung bei direkt rauchgasbefeuerten Trocknern ermöglicht.

2.2 Die Lösung besteht darin, daß das Verfahren bei Trocknungsprozessen insbesondere in Textiltrocknern, unter Verwendung mindestens eines Sauerstoffsensors, vorzugsweise auf der Basis einer Zirkoniumdioxidzelle, geschieht, wobei in durch Verbrennungsgase (Rauchgase) direkt beheizten Trocknungsprozessen der Sauerstoffgehalt der Abluft des Trocknungsprozesses und zusätzlich eine weitere, die Rauchgaszusammensetzung nach der Verbrennungsrechnung bestimmende Komponente gemessen werden.

2.3 Anwendung des Verfahrens insbesondere bei Wärmebehandlungsprozessen, beispielsweise Trockenprozessen.

Fig. 1

Die Erfindung betrifft ein Verfahren zur Bestimmung der Beladung von Luft mit Dämpfen, insbesondere Wasserdampf, bei Trocknungsprozessen in Trocknern, insbesondere Textiltrocknern, unter Verwendung mindestens eines Sauerstoffsensors auf der Basis einer Zirkoniumdioxydzelle.

Verfahren dieser Art sind für verschiedenste Trocknungsprozesse bzw. Wärmebehandlungsverfahren beispielsweise in der Textil-, Papier- oder Nahrungsmittelindustrie erforderlich, um eine Optimierung dieser Verfahren insbesondere hinsichtlich Trocknungsgrad, Qualität der behandelten Produkte und wirtschaftlichem Energieeinsatz durchzuführen.

Es sind zahlreiche unterschiedliche Verfahren zur Messung der Luftfeuchte bekannt (vgl. Lück "Feuchtigkeit", Verlag Oldenbourg München/Wien), die insbesondere bei der Anwendung in Trocknern bei höheren Temperaturen und bei Staub sowie kondensierbaren Bestandteilen in der Trocknungsluft zu Schwierigkeiten und unzureichend genauen Ergebnissen führen können. Bei bekannten Verfahren (vgl. DE-OS 22 35 853 bzw. DE-OS 28 56 288) wird ein Meßgasstrom mit Hilfe eines Kühlers unter den Taupunkt abgekühlt, die im Kühler abgegebene Wärmeleistung sowie die Temperatur des Meßgases vor und nach der Abkühlung werden gemessen und der Feuchtegehalt des Meßgases durch Auswertung der Wärmebilanzgleichung bestimmt.

Nachteilig bei dieser bekannten Verfahrensweise ist, daß ein Teilstrom als Meßgas entnommen werden muß, wobei die Verschmutzung der Entnahmeleitung durch aufwendige Maßnahmen verhindert werden muß. Dies ist besonders schwierig, wenn bei Trocknungsprozessen Kohlenwasserstoffe, beispielsweise Harze, Avivagen, freigesetzt werden. Probleme dieser Art sind bei Einsatz von Einbausonden, z.B. auf der Basis von Zirkoniumdioxidzellen, wie sie als "Lambda-Sonden" zur Verbesserung der Verbrennung im Abgas von Automobilen Verwendung finden, nicht vorhanden.

Es ist bekannt, solche Sonden unter Anwendung des Dalton'schen Gesetzes zur Feuchtebestimmung einzusetzen (vgl. YEW-Druckschrift GS 11M5A1-E, 1986). Ihr Einsatz bei Trocknern ist jedoch auf solche beschränkt, bei denen die Zuluft zum Trockenprozeß ca. 21 % Sauerstoff (Volumenanteil) hat, wie dies bei indirekt beheizten Trocknern der Fall ist.

Es ist Aufgabe der Erfindung, ein Verfahren anzugeben, das die Anwendung von Sauerstoffsensoren zur Feuchtemessung bei direkt Rauchgas befeuerten Trocknern ermöglicht. Das Verfahren soll weiterhin so ausgestaltet werden, daß in kombinierten Wärmebehandlungsprozessen, insbesondere Trocknungsprozessen, austretende schädliche Gase, sogenannte Avivagen, zusammen mit dem Wasserdampfanteil zwecks optimaler Regelung des Trocknungsprozesses erfaßt werden.

Diese Aufgabe wird dadurch gelöst, daß in durch Verbrennungsgase (Rauchgase) direkt beheizten Trocknungsprozessen der Sauerstoffgehalt der Abluft des Trocknungsprozesses und zusätzlich eine weitere, die Rauchgaszusammensetzung nach der Verbrennungsrechnung bestimmende Komponente gemessen werden. Dabei kann vorteilhaft als zusätzliche Komponente die Temperatur der Rauchgase unmittelbar nach der Verbrennung bestimmt werden. Es ist aber auch möglich, anstelle der Temperatur der Rauchgase als Zusatzkomponente den Kohlendioxidanteil und/oder den Sauerstoffanteil der Rauchgase zu bestimmen, wobei die Kohlendioxidmessung am Ort der Sauerstoffmessung in der Abluft des Trocknungsprozesses, also hinter dem Trocknungsprozeß, erfolgt und aus dem Verhältnis von Kohlendioxid zu Sauerstoff nach der Verbrennungsrechnung die Rauchgaszusammensetzung bestimmt wird. Grundsätzlich erfolgt sonst die zur Sauerstoffmessung zusätzliche Messung, nämlich die Messung der Temperatur der Rauchgase, die Messung des Sauerstoffgehaltes und/oder die Messung des Kohlendioxides in der Zuluft zum eigentlichen Trocknungsprozeß, also vor der Bildung von Wasserdampf bzw. Schadgasen beim Trocknungsprozeß. Die zusätzlich zu erfassende Komponente wird in der Regel kontinuierlich gemessen, sie kann aber auch durch eine einmalige Einstellung für den stationären Betriebszustand des Trockners festgelegt werden. Die Flammtemperatur des Brenners kann beispielsweise mit einem Bolometer gemessen werden. Die Verbrennungsrechnung ergibt dann den Faktor, der zur Umrechnung entsprechend der nachstehend angegebenen Gleichung zu verwenden ist. Bei kontinuierlicher Messung ist vorteilhaft in der Software der Auswerteelektronik eine Tabelle für die Verbrennungsrechnung abgelegt. Nachstehend ist hierfür ein Beispiel angegeben. Voraussetzung ist dabei, daß die Brennstoffzusammensetzung oder der Heizwert des Brennstoffes bekannt sind.

Die Erfindung wird im folgenden näher erläutert. Es wird zunächst die Gleichung angegeben, nach der die Umrechnung von Sauerstoffvolumenanteil in Wasserdampfvolumenanteil zu erfolgen hat.

$$V_{H2O}/V = 1 - (V_{O2}/V) \times \{1 + (V_{0*}/V_0) / (V_{0O2}/V_0)\}$$

Diese Gleichung läßt sich durch Anwendung des Dalton'schen Gesetzes ableiten. In ihr bedeuten:

V =             Meßvolumen (Gesamtvolumen) in der Abluft, also nach dem Trocknungsprozeß;

2

$V_{H2O}$ = Wasserdampfteilvolumen im Meßvolumen;

$V_{O2}$ = Sauerstoffteilvolumen im Meßvolumen;

$V_0$ = Zuluftvolumen vor "Addition" von Wasserdampf, also vor dem Trocknungsprozeß;

$V_{0*}$ = Teilvolumen der "Komplementärgase " (Zuluftvolument abzüglich Sauerstoff- und Wasserdampfvolumen) im Zuluftvolumen;

$V_{0O2}$ = Teilvolumen des Sauerstoffs im Zuluftvolumen.

Da die gesamte Beladung der Luft erfaßt wird, werden außer Wasserdampf auch andere beim Wärmebehandlungsprozeß entstandenen Dämpfe erfaßt, beispielsweise schädliche Gase, wie verdampfte Avivagen bei Trocknungsprozessen von Textilien, die zu dem Zuluftvolumen infolge des Trocknungsprozesses hinzukommen und demgemäß in der Abluft hinter dem Wärmebehandlungsprozeß (z.B. Trocknungsprozeß) enthalten sind.

Der Sauerstoffsensor, vorzugsweise in Form einer Sauerstoffsonde mit Zirkoniumdioxid-Elektrode, gibt ein von $(V_{O2}/V)$ abhängiges Nutzsignal ab. Der Zusammenhang ist durch die Nernst'sche Gleichung gegeben.

Bei direkt beheizten Trocknern wird die Zusammensetzung und der Zustand der Zuluft $V_0$ in der nachstehenden beispielhaften Tabelle für Beheizung mit leichtem Heizöl tabelliert. Zur Festlegung genügt ein einziger Parameter. Vorzugsweise wird die Zulufttemperatur bestimmt. Diese Messung kann kontinuierlich erfolgen oder bei stationären Brennerverhältnissen einmalig bei Betriebnahme. Es ist selbstverständlich möglich, das Verfahren auch zur begrenzten Rauchgasanalyse anzuwenden, wobei aus Sauerstoffanteil und Rauchgastemperatur der Kohlendioxidanteil bestimmt werden kann. Zur Aufstellung nachstehender Tabelle benötigt man die Brennstoffzusammensetzung oder mindestens den Heizwert des Brennstoffes. Im letzteren Fall läßt sich eine ausreichende Näherungsrechnung durchführen (vgl. hierzu FDBR-Handbuch, Wärme- und Strömungstechnik).

Bei Trocknungs- oder Wärmebehandlungsprozessen, die keine hohen Temperaturen vertragen, gibt es zwei Möglichkeiten, nämlich einmal, den Brenner mit hohem Luftüberschuß zu fahren, um die Rauchgastemperatur und damit die Zulufttemperatur für den eigentlichen Trocknungsprozeß zu senken oder die Außenluft unter Umgehung des Brenners dem Rauchgas zuzumischen, wodurch die Zuluft zum Trocknungsprozeß ebenfalls entsprechend gesenkt wird. In diesem Fall kann man mit weniger Luftüberschuß fahren.

Bei indirekt beheizten Trocknern ist die Zuluft $V_0$ die Außenluft (Umgebungsluft). Trockene Außenluft hat einen $O_2$-Volumenanteil von 0,21. Der Ausdruck in der geschweiften Klammer ist dann 4,76 (= 1/0,21).

## Tabelle für leichtes Heizöl

Heizwert $H_U$ = 42,5 MJ/kg

Statistische Analyse: (Masse %)

C: 85,7 %   H: 12,7 %   S: 0,9 %   N: 0 %   O: 0 %   N+O %: 0,7 %

| Luftüber-schußzahl | Rauchgas-temperatur t | Sauerstoff $O_2$ | Kohlen-dioxid $CO_2$ | Wasser-dampf $H_2O$ | $O_2/CO_2$ { } | |
|---|---|---|---|---|---|---|
| (-) | (°C) | (Vol%) | (Vol%) | (Vol%) | (-) | (-) |
| 1,2 | 1860 | 3,2 | 11,4 | 10,9 | 0,29 | 27,84 |
| 1,6 | 1478 | 7,5 | 8,6 | 8,5 | 0,87 | 12,20 |
| 2,0 | 1238 | 10,1 | 7,0 | 7,0 | 1,44 | 9,21 |
| 2,5 | 1021 | 12,2 | 5,6 | 5,8 | 2,18 | 7,72 |
| 3,0 | 874 | 13,6 | 4,7 | 5,0 | 2,89 | 6,99 |
| 3,5 | 765 | 14,6 | 4,0 | 4,4 | 3,65 | 6,55 |
| 4,0 | 680 | 15,4 | 3,5 | 4,0 | 4,40 | 6,23 |
| 5,0 | 558 | 16,4 | 2,8 | 3,4 | 5,85 | 5,89 |
| 7,5 | 386 | 17,9 | 1,9 | 2,6 | 9,42 | 5,44 |
| 10,0 | 296 | 18,6 | 1,4 | 2,1 | 13,29 | 5,26 |
| 15,0 | 203 | 19,3 | 1,0 | 1,7 | 19,30 | 5,09 |
| 20,0 | 155 | 19,7 | 0,7 | 1,5 | 28,14 | 5,00 |

Die Tabelle zeigt, daß man zur Bestimmung der Zuluftzusammensetzung auch den Sauerstoffanteil oder den Kohlendioxidanteil der Zuluft verwenden kann.

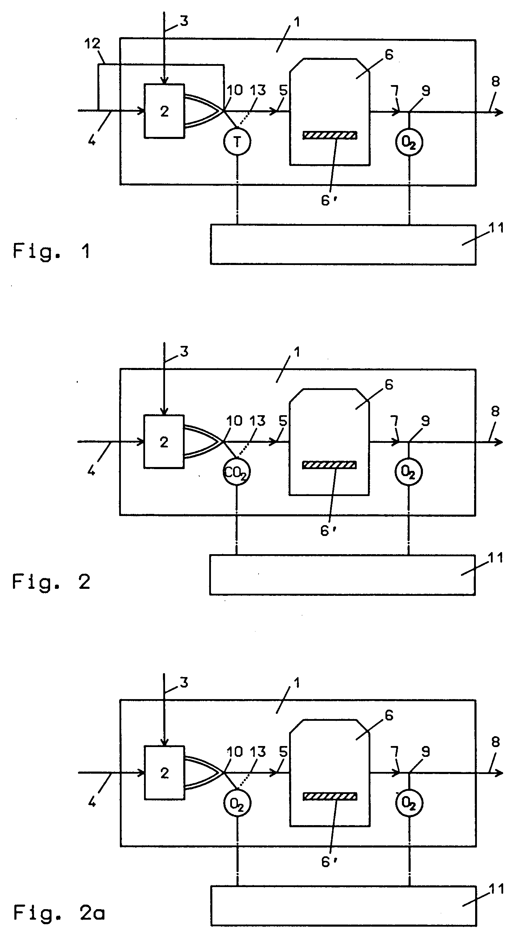

Das erfindungsgemäße Verfahren wird nachstehend anhand schematisch dargestellter Ausführungsbeispiele ergänzend erläutert. In Fig. 1 ist mit 1 der Trockner bezeichnet, in welchem sich zur direkten Beheizung der Brenner 2 befindet. Diesem wird über 3 das Heizmedium, beispielsweise leichtes Heizöl, und über 4 Außenluft zugeführt. Die vom Brenner abströmenden Rauchgase gehen als Zuluft entsprechend Pfeil 5 in den eigentlichen Trocknungsprozeß 6 oder einen anderen Wärmebehandlungsprozeß ein, bei dem das zu trocknende Gut, z.B. eine Textilbahn 6', getrocknet wird. Während des Trocknungsprozesses wird die Zuluft mit Wasserdampf und/oder anderen gasförmigen Schadstoffen (Avivagen) beladen und verläßt den Trocknungsprozeß als Abluft (vgl. Pfeil 7). Die aus dem Trockner 1 austretende Abluft wird als Fortluft (vgl. Pfeil 8) bezeichnet und je nach Betriebszuständen im Bedarfsfall in ihrer Menge geregelt.

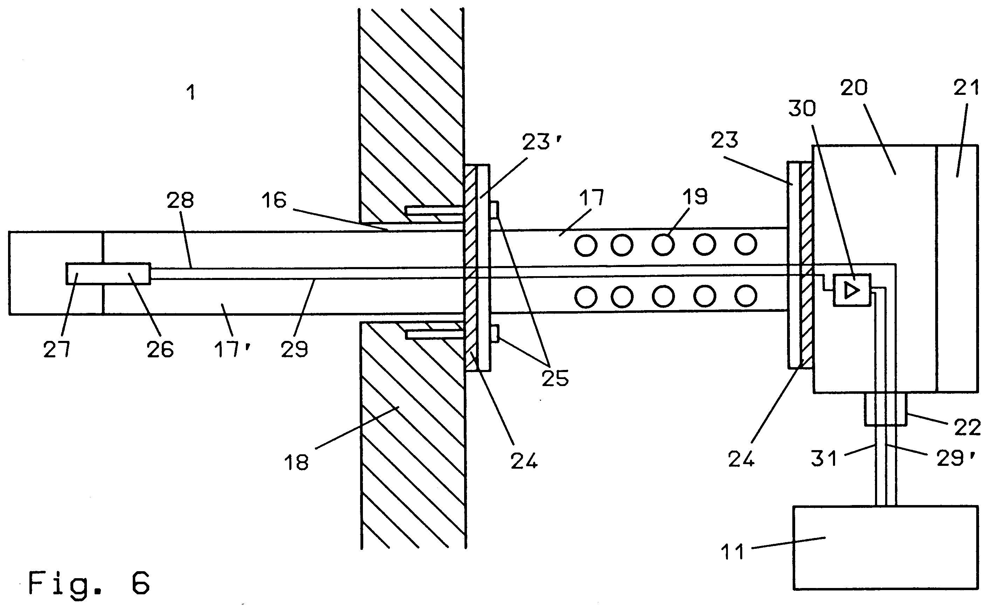

Der Sauerstoffgehalt der Abluft des Trockenprozesses wird in der den Trocknungsprozeß 6 verlassenden Abluft bei 9 gemessen. Hierzu dient die in Fig. 6 schematisch dargestellte Meßsonde. Die zusätzlich zu messende Komponente zur Bestimmung der Rauchgaszusammensetzung nach der Verbrennungsrechnung ist in der Fig. 1 die Rauchgastemperatur, die unmittelbar nach der Verbrennung bei 10 bestimmt wird. Der bei 9 gemessene Sauerstoffwert und der bei 10 gemessene Temperaturwert werden einer Auswerteelektronik 11 auf Mikroprozessorbasis mit Software für Verbrennungsrechnung zugeführt.

Mit 12 ist eine Bypass-Leitung dargestellt, mittels der ein Teilstrom der Außenluft unter Umgehung des Brenners 2 den Rauchgasen unmittelbar bei Austritt aus dem Brenner, und zwar vor der Temperaturmeßstelle 10 zugeführt wird. Diese Bypass-Leitung 12 kann bei den übrigen Ausführungsbeispielen gleichfalls vorgesehen werden.

Die Sauerstoffsonde bei 9 mißt den relativen Sauerstoffanteil im Meßvolumen, nämlich in der Abluft oder Umluft, der infolge Austretens von Dämpfen aus dem zu trocknenden Gut 6' niedriger ist als in der zum Trocknungsprozeß 6 strömenden Zuluft, wobei die Dämpfe sich aus Wasserdampf und bei entsprechend höheren Trocknungstemperaturen zusätzlich noch aus schädlichen Gasen (Avivagen) zusammensetzen.

Anstelle der Temperatur der Abgase wird in Fig. 2 der $C_{O2}$-Gehalt der Zuluft bei 13 gemessen.

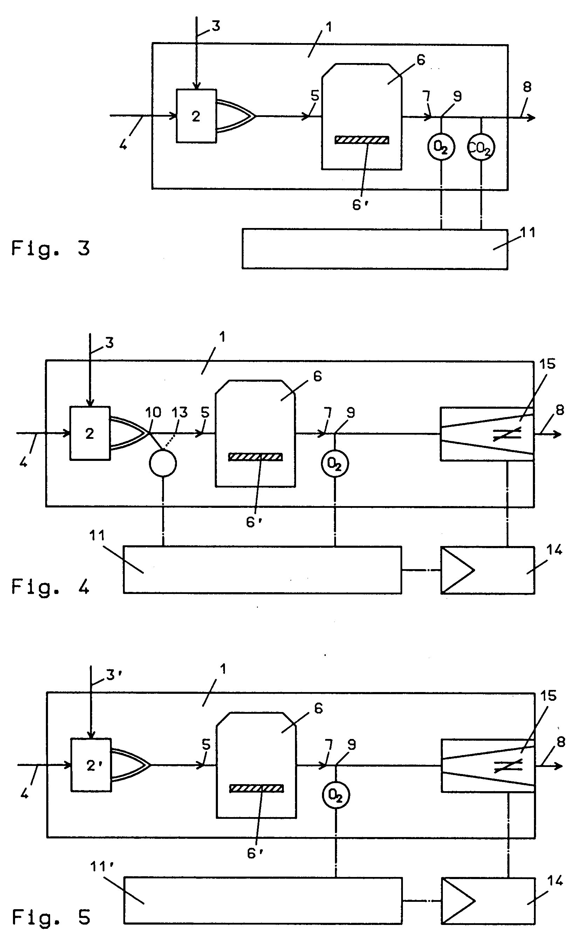

In Fig. 2a wird unmittelbar im Bereich des Austrittes der Rauchgase aus dem Brenner 2 anstelle der Temperatur der Rauchgase als zusätzliche Komponente der Sauerstoffgehalt der Zuluft gemessen. Fig. 3 zeigt die grundsätzlich gleiche Meßanordnung wie Fig. 2, bei der die zusätzliche Komponente Kohlendioxid anstatt in der Zuluft entsprechend Fig. 2 am gleichen Ort wie der Sauerstoff, nämlich in der Abluft zwecks Auswertung des Volumenverhältnisses von Kohlendioxid zu Sauerstoff gemessen wird. In den Fig. 1 bis 2a ist eine weitere mögliche Meßstelle 13 eingezeichnet; sie kann zur Messung der Temperatur der Rauchgase oder des Kohlendioxidgehaltes bzw. des Sauerstoffgehaltes vor dem Trocknungsprozeß liegen.

Alle Ausführungsbeispiele der Fig. 1 bis 3 lassen sich bei reinen Trocknungsverfahren, insbesondere aber auch mit Vorteil bei kombinierten Verfahren, anwenden, bei denen außer Wasserdampf zusätzlichs Gase, wie Schadstoffe, Avivagen oder dgl., die Abluft beladen. Dies ist besonders bei den heute immer mehr zur Anwendung kommenden Produktionsverfahren, beispielsweise Trocknen und Fixieren in einem Arbeitsgang, Thermofixieren von bereits trockenen Textilwaren, Kondensieren und Thermosolieren, von Bedeutung, da bei diesen Prozessen die Umluft außer mit Wasserdampf noch mit zusätzlichen, meist schädlichen Gasen beladen wird. Die bisherigen Meßmethoden waren hierfür ungeeignet, da mit ihnen diese Gase nicht erfaßt werden können.

Zur Verringerung der Arbeitsplatzbelastung und zur Vermeidung der Verschmutzung von Trockner und Rohrleitungen durch kondensierende Schadstoffe, beispielsweise Spinnöle bei Textilien (Avivagen), ist es erforderlich, die Fortluftmenge entsprechend der Beladung der Abluft zu regeln. Beispielsweise bei hohem Anteil der Schadstoffe größere Fortluftmengen zu fahren als sie bei einem reinen Trocknungsprozeß erforderlich wären. Die Einstellung dieser größeren Fortluftmenge erfolgt bisher von Hand, und zwar nach Gefühl der Bedienungsperson. Dabei ist die Fehlerquote für das Auffinden der richtigen Einstellung sehr hoch. Bei einmal gefundener richtiger Einstellung der Fortluftmenge ist bei Vergrößerung der Warengeschwindigkeit die Fortluftmenge meist zu gering und bei kleinerer Warengeschwindigkeit bzw. bei Maschinenstillstand zu groß. Da bei zu großer Fortluftmenge auch die aufzuheizende Außenluft zu groß wird, steigt entsprechend der Energieverbrauch.

Mit dem erfindungsgemäßen Verfahren kann somit nicht nur Wasserdampf als zusätzliche Komponente zur Zuluft $V_O$, sondern die Gesamtbeladung einschließlich etwaiger Schadstoffe gemessen werden. Dadurch ist es möglich, die Fortluftmenge in Abhängigkeit von der gesamten Beladung (Wasserdampf und schädliche Gase) zu regeln.

In Fig. 4 ist schematisch eine Regelung bei direkter Beheizung des Trockners dargestellt, wobei dieselben Meßanordnungen, wie in den Fig. 1 bis 3 gezeigt, anwendbar sind. Der mittels der Auswerteelektronik 11 festgestellte Meßwert der Beladung der Luft mit Wasserdampf und/oder zusätzlichen Gasen wird als Regelgröße einem Regler 14 zugeführt, der die Menge der Fortluft 8 regelt, wobei die Regelung über Jalousieklappen oder drehzahlregelbare Gebläse 15 erfolgen kann.

Die der Erfindung zugrundeliegende Erfassung der Schadstoffe kann auch zur Regelung der Fortluftvolumenströme von indirekt beheizten Trocknern, insbesondere Textiltrocknern, angewandt werden. Hierzu wird aus der Messung des Verhältnisses des Sauerstoffgehaltes der Abluft zum Sauerstoffgehalt der Zuluft nach dem Trocknungsprozeß die gesamte Beladung der Luft mit Wasserdampf und/oder schädlichen Gasen bei nicht reinem Trocknungsprozeß ermittelt und als Regelgröße ebenfalls einem Fortluftmengenregler 15 zugeführt, wie dies in Fig. 5 schematisch gezeigt ist; dabei ist mit 2' ein im Trockner 1 vorgesehener Wärmetauscher oder dgl. bezeichnet, dem über 3' das Heizmedium, z.B. heißes Gas, und über 4 die aufzuwärmende Außenluft zugeführt wird.

In der Fig. 6 ist die bei dem erfindungsgemäßen und bei anderen Verfahren einsetzbare Meßsonde schematisch gezeichnet. Sie dient zur Messung des Sauerstoffgehaltes in der Abluft, wie dies in den Fig. 1 bis 5 schematisch dargestellt ist. Die Meßsonde weist einen außerhalb des Trockners 1, also außerhalb der Trocknerwand 18, angeordneten Rohrteil 17 mit Öffnungen 19 für den Eintritt der Umgebungsluft auf. Dieses Rohrteil 17 trägt an seinem freiliegenden Ende ein Meßvorverstärkergehäuse 20 mit Deckel 21, in welchem der Vorverstärker vorgesehen ist. Mit 22 ist eine Kabeldurchführung nach außen bezeichnet. Das Meßvorverstärkergehäuse 20 ist im Ausführungsbeispiel mittels Flansch 23 am Rohr 17 befestigt, das an der Trocknerwand mittels Flansch 23' unter Zwischenschaltung einer Wärmedämm- und Abdichtplatte 24 mittels Schrauben 25 in der Weise befestigt, daß ein Luftspalt 16 verbleibt. Der in den Trockner 1 hineinragende Abschnitt 17' des Rohres 17 hat im Bereich seines freien Endes den Zirkoniumdioxidsensor, wobei dessen Innenelektrode 26 von der Außenelektrode 27 gasdicht getrennt ist. Die Innenelektrode 26 des Sauerstoffsensors ist der Referenzluft, also der Umgebungsluft, die 21 Vol% Sauerstoff enthält, ausgesetzt, während die Außenelektrode 27 von der Abluft nach dem Trocknungsprozeß beaufschlagt wird.

Wesentlich ist nun bei der erfindungsgemäßen Meßsonde, daß die Öffnungen 19 im Mantel des äußeren Rohrteils 17 nach Form und Größe so ausgebildet sind, daß sie einen optimalen, freien Eintritt der Außenluft, also der Referenzluft, in das Innere der Meßsonde gewährleisten und andererseits aber den Wärmedurchgangsquerschnitt des Rohrteiles 17 zu dem an seinem Außenende angeordneten Meßverstärker 20 derart vermindern, daß dieser weitgehend von den hohen Temperaturen (beispielsweise 300 °C bis 400 °C) des die Elektroden 26, 27 tragenden Rohrteiles 17' abgeschirmt ist. Um eine größtmögliche Kühlwirkung für den Meßverstärker zu erreichen, ist eine Mehrzahl einzelner, voneinander getrennter Öffnungen 19 über einen ausreichend großen Längenabschnitt des Rohrteiles 17 vorgesehen. Hierdurch kann die Außenluft auf einem verhältnismäßig großen Abschnitt allseits und gleichmäßig als Referenzluft in die Rohrabschnitte 17, 17' gelangen. Vorteilhaft sind dabei die Öffnungen 19 als runde Löcher ausgebildet und gleichmäßig über den Umfang des Rohrteiles 17 verteilt angeordnet. Hierdurch ergeben sich vor dem Gehäuse 20 des Meßverstärkers stegartige Materialquerschnitte, die den Wärmefluß zum Meßverstärker reduzieren und ihn dadurch kühl halten.

Nach einem weiteren Merkmale der Erfindung wird die Meßspannung gemäß der Nernst'schen Gleichung auf einen durch den Meßvorverstärker 20 eingeprägten Strom verstärkt. Hierzu ist im Gehäuse 20 ein Vorverstärker 30 vorgesehen, der das von der Zirkoniumdioxidelektrode 26, 27 kommende Meßsignal 29 verstärkt, das dann als vorverstärktes Meßsignal 29' der Auswerteelektronik zugeleitet wird. Das Meßsignal und die Heizspannung 28 werden durch Gehäuse und die Rohrabschnitte 17, 17' zur Meßsonde 26 geführt. Durch den vorgesehenen Vorverstärker können beliebig lange Leitungen benutzt werden. Infolge der vorgesehenen wärmeabschirmenden Ausbildung der Löcher 19 wird dabei der Vorverstärker in erwünschter Weise stets kühl gehalten.

Da Sauerstoffsensoren Toleranzen unterliegen, wird im Vorverstärker 30 eine Abgleichmöglichkeit in Form eines Kalibriersignales 31 vorgesehen. Dieses Kalibriersignal ist ein Maß für die Charakteristik des Sauerstoffsensors. Es ermöglicht in einfacher und sehr vorteilhafter Weise eine Austauschbarkeit von kompletten Sonden, ohne daß beim Austausch dieser Sonden neue Vergleichsmessungen oder Eichungen vorgenommen werden müssen. Wie aus Fig. 6 ersichtlich, geht das Kalibriersignal 31 ebenso wie das Vorverstärkersignal 30 auf die Auswerteelektronik 11 auf Mikroprozessorbasis.

Für Messungen an direkt beheizten Trocknern (Fig. 1 bis 4) ist an der Auswerteelektronik 11 die Heizmittelzusammensetzung oder wenigstens der Heizwert des über Leitung 3 zugeführten Heizmittels (Gas oder leichtes Heizöl) über eine geeignete Kodierung eingebbar. Die Temperatur des Rauchgases kann von der Auswerteelektronik als kontinuierliches Signal oder über eine Kodierung als einmalig eingestelltes Signal ausgewertet werden.

**Patentansprüche**

1. Verfahren zur Bestimmung der Beladung von Luft mit Dämpfen, insbesondere Wasserdampf, bei Trocknungsprozessen insbesondere in Textiltrocknern, unter Verwendung mindestens eines Sauerstoffsensors, insbesondere eines Sauerstoffsensors auf der Basis einer Zirkoniumdioxidzelle,
dadurch gekennzeichnet, daß in durch Verbrennungsgase (Rauchgase) direkt beheizten Trocknungsprozessen der Sauerstoffgehalt der Abluft des Trocknungsprozesses und zusätzlich eine weitere, die Rauchgaszusammensetzung nach der Verbrennungsrechnung bestimmende Komponente gemessen werden.

2. Verfahren nach Anspruch 1,
dadurch gekennzeichnet, daß als zusätzliche Komponente die Temperatur der Rauchgase unmittelbar nach der Verbrennung bestimmt wird (Fig. 1).

3. Verfahren nach Anspruch 1,
dadurch gekennzeichnet, daß als zusätzliche Komponente der Kohlendioxidanteil der Rauchgase und/oder ihr Sauerstoffanteil bestimmt werden (Fig. 2, 2a).

4. Verfahren nach Anspruch 1,
dadurch gekennzeichnet, daß die Kohlendioxidmessung am Ort der Sauerstoffmessung in der Abluft des Trocknungsprozesses erfolgt (Fig. 3) und aus dem Verhältnis von Kohlendioxid zu Sauerstoff nach der Verbrennungsrechnung die Rauchgaszusammensetzung bestimmt wird.

5. Verfahren nach einem der Ansprüche 1 bis 4,
dadurch gekennzeichnet, daß die Messungen der Rauchgastemperatur in der Zuluft vor dem Trocknungsprozeß erfolgt (Fig. 1).

6. Verfahren nach Anspruch 3,
dadurch gekennzeichnet, daß die Kohlendioxidmessung in der Zuluft vor dem Trocknungsprozeß erfolgt (Fig. 2).

7. Verfahren nach Anspruch 4,
dadurch gekennzeichnet, daß die zusätzliche Messung des Sauerstoffgehaltes in der Zuluft vor dem Trocknungsprozeß erfolgt (Fig. 2a).

8. Verfahren nach einem der Ansprüche 1 bis 7,
dadurch gekennzeichnet, daß die Messungen der zusätzlichen Komponenten kontinuierlich vorgenommen werden.

9. Verfahren nach einem der Ansprüche 1 bis 8,
dadurch gekennzeichnet, daß die Sauerstoffmessung und die Messung einer der zusätzlichen Komponenten in eine Auswerteelektronik gegeben werden, in welcher die Beladung der Luft mit Dämpfen des Trockenprozesses nach der Dalton'schen Gleichung errechnet wird.

10. Verfahren nach Anspruch 9,
dadurch gekennzeichnet, daß in das Programm der Auswerteelektronik die Gleichung:

$$V_{H2O}/V = 1 - (V_{O2}/V) \times \{1 + (V_{0*}/V_0) / (V_{0O2}/V_0)\}$$

eingegeben wird;

hierin bedeuten:

$V$ = Meßvolumen (Gesamtvolumen) in der Abluft, also nach dem Trocknungsprozeß;
$V_{H2O}$ = Wasserdampfteilvolumen im Meßvolumen;
$V_{O2}$ = Sauerstoffteilvolumen im Meßvolumen;
$V_0$ = Zuluftvolumen vor "Addition" von Wasserdampf, also vor dem Trocknungsprozeß;
$V_{0*}$ = Teilvolumen der "Komplementärgase" (Zuluftvolument abzüglich Sauerstoff- und Wasserdampfvolumen) im Zuluftvolumen;
$V_{0O2}$ = Teilvolumen des Sauerstoffs im Zuluftvolumen.

11. Verfahren nach einem der Ansprüche 1 bis 10,
dadurch gekennzeichnet, daß der mittels der Auswerteelektronik festgestellte Meßwert der Beladung der Luft mit Wasserdampf und/oder schädlichen Gasen (Avivagen) bei Trocknungsprozessen als Regelgröße einem die den Trockner verlassende Fortluftmenge regelnden Regler zugeführt wird (Fig. 4).

12. Verfahren zur Regelung der Fortluftvolumenströme von indirekt beheizten Trocknern, insbesondere Textiltrocknern,
dadurch gekennzeichnet, daß aus der nach dem Trocknungsprozeß vorgenommenen Messung des Verhältnisses des Sauerstoffgehaltes der Abluft zum Sauerstoffgehalt der Zuluft die gesamte Beladung der Luft mit Wasserdampf bzw. schädlichen Gasen bei nicht reinem Trocknungsprozeß ermittelt und als Regelgröße einem Fortluftmengenregler zugeführt wird (Fig. 5).

13. Vorrichtung zur Bestimmung der Beladung von Luft mit Dämpfen, insbesondere nach einem der Ansprüche 1 bis 12, mittels einer rohrförmigen Meßsonde, die einen von den zu messenden Gasen beaufschlagten Rohrteil (17') sowie einen Zirkoniumdioxidsensor mit einer Innen- und einer Außenelektrode (26, 27) aufweist, während ein zweiter Rohrteil (17) der Außenluft derart ausgesetzt ist, daß diese in die Rohrteile (17, 17') einströmt und die Innenelektrode (26) beaufschlagt und durch das Rohr (17, 17') der Meßsonde elektrische Leiter geführt sind,
dadurch gekennzeichnet, daß der Mantel des äußeren Rohrteils (17) Öffnungen (19) hat, die nach Form und Größe einen optimal freien Eintritt der Referenzluft in das Innere der Meßsonde (17, 17') gewährleisten und andererseits den Wärmedurchgangsquerschnitt des Rohrteiles (17) zu einem an seinem äußeren Ende angeordneten Meßverstärkergehäuse (20) zwecks Kühlhaltung desselben verkleinern.

14. Vorrichtung nach Anspruch 13,
dadurch gekennzeichnet, daß eine Mehrzahl einzelner und voneinander getrennter Öffnungen (19) über

7

einen längeren Abschnitt des Rohrteiles (17) angeordnet sind.

15. Vorrichtung nach Anspruch 13 oder 14,
dadurch gekennzeichnet, daß die Öffnungen (19) als runde Löcher ausgebildet und gleichmäßig über den Umfang des Rohrteiles (17) verteilt sind.

16. Vorrichtung nach einem der Ansprüche 13 bis 15,
dadurch gekennzeichnet, daß der Meßverstärker ein Gehäuse (20) hat, in welchem ein Vorverstärker (30) für das von der Elektrode (26, 27) kommende Meßsignal (29) vorgesehen ist, und daß eine Leitungsverbindung (29') für das Vorverstärkermeßsignal zur Auswerteelektronik (11) vorgesehen ist.

17. Vorrichtung nach einem der Ansprüche 13 oder 16,
dadurch gekennzeichnet, daß in den Vorverstärker (30) ein Kalibriersignal integriert ist, welches das Maß für die Charakteristik des Sauerstoffsensors bildet.

Fig. 1

Fig. 2

Fig. 2a

Fig. 3

Fig. 4

Fig. 5

Fig. 6